(19) Europäisches Patentamt
European Patent Office
Office européen des brevets

(11) Veröffentlichungsnummer: **0 217 214 B1**

# (12) EUROPÄISCHE PATENTSCHRIFT

(45) Veröffentlichungstag der Patentschrift: **02.05.90**

(51) Int. Cl.⁴: **C07D 487/04**, C09B 57/00, C08K 5/34 // (C07D487/00, 209:00, 209:00)

(21) Anmeldenummer: **86112721.5**

(22) Anmeldetag: **15.09.86**

(54) **2,4-Dioxopyrrolo(2,3-c)pyrrole.**

(30) Priorität: **24.09.85 DE 3533949**

(43) Veröffentlichungstag der Anmeldung: **08.04.87 Patentblatt 87/15**

(45) Bekanntmachung des Hinweises auf die Patenterteilung: **02.05.90 Patentblatt 90/18**

(84) Benannte Vertragsstaaten: **CH DE FR GB IT LI**

(56) Entgegenhaltungen:
**EP-A- 0 094 911**
**EP-A- 0 133 156**

**Journal of the Chemical Society 1958 V.M. Clark et al. "Chemistry of the Vitamin B12 Group. Part VII. The Products of Chromic Acid Oxidation" Seiten 3283-3289**
**Tetrahedron Letters 1974 (25) G. Singh et al. "Synthesis of a mesoionic Imidazole System and Studies of its Participation in 1:3 dipolar Cycloaddition Reactions" Seiten 2169-2170**

(73) Patentinhaber: **BAYER AG, D-5090 Leverkusen 1 Bayerwerk(DE)**

(72) Erfinder: **Fürstenwerth, Hauke, Dr., Morgengraben 3, D-5000 Köln 80(DE)**
Erfinder: **Rolf, Meinhard, Dr., Berta-von-Suttner-Strasse 24, D-5090 Leverkusen(DE)**

**EP 0 217 214 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Aus der EP-A 0 133 156 sind substituierte 1,4-Diketo-pyrrolo[3,4-c]-pyrrole bekannt. Die Erfindung betrifft 2,4-Dioxopyrrolo[2,3-c]-pyrrole, die in einer ihrer tautomeren Formen der Formel

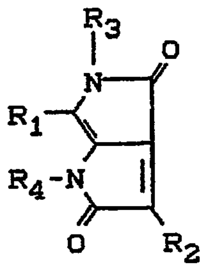

(I)

entsprechen, in der

$R_1$, $R_2$ Phenyl, Diphenlyl, Naphthyl oder einen heterocyclisch-aromatischen Rest, der 1, 2, 3 oder 4 fünf-, sechs- oder siebengliedrige Ringe enthält, von denen mindestens einer 1, 2 oder 3 Heteroatome aus der Reihe N, O, S enthält, bezeichnen, wobei die für $R_1$, $R_2$ genannten Reste durch Halogen, $-CN$, $-R_5$, $OR_6$, $SR_6$, $-N(R_7)(R_8)$, $-COOR_9$, $-N(R_{10})COR_9$, $-N(R_6)COOR_9$, $N(R_6)$-$CON(R_7)(R_8)$, $-NHSO_2R_9$, $-SO_2R_9$, $-SOR_9$, $-SO_2OR_9$, $-CON(R_7)(R_8)$, $-SO_2N(R_7)(R_8)$, $-N{=}N-R_{11}$, $-OCOR_9$ und $-OCONHR_9$ substituiert sein können,

$R_3$, $R_4$ für Wasserstoff, $C_2$–$C_{18}$-Alkenyl und $C_2$–$C_{18}$-Alkinyl, die durch Halogen, $-CN$, $-OCOR_9$, $-OR_7$, $-COOR_9$, $-SR_7$, $-CON(R_7)(R_8)$, $-OCONHR_9$ substituiert sein können, Epoxyethyl, Phenyl und Naphthyl, die durch F, Cl, Br, $-NO_2$, $-CN$, $-CF_3$, $C_1$–$C_{18}$-Alkyl, $C_1$–$C_{18}$-Alkoxy, Phenyl, Phenoxy, $C_1$–$C_6$-Alkoxycarbonyl und Phenoxycarbonyl substituiert sein können, $-R_5$, $-COR_6$,

$$-CON(R_7)(R_8),\ \underset{\displaystyle R_{12}}{\underset{|}{-C}}{=}A$$ oder einen Rest

der Formel

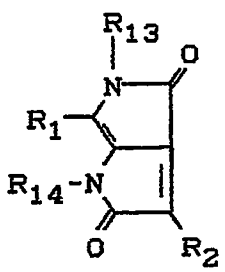

(Ia)

stehen und

$R_5$ $C_1$–$C_{18}$-Alkyl oder $C_3$–$C_7$-Cycloalkyl, die durch Halogen, $-CN$, $-OCOR_9$, $-OR_7$, $-COOR_9$, $-SR_7$, $-CON(R_7)(R_8)$ und $-OCONHR_9$ substituiert sein können,

$R_6$, $R_7$, $R_8$ Wasserstoff, $C_1$–$C_{18}$-Alkyl, Cyclopentyl, Cyclohexyl, die durch Cl, Br, F, $-CN$, $-OH$, Mono-$C_1$–$C_4$-alkylamino, Di-$C_1$–$C_4$-alkylamino, gegebenenfalls durch Cl, Br, F, $C_1$–$C_6$-Alkyl und $C_1$–$C_6$-Alkoxy substituiertes Phenyl oder Naphthyl, oder heterocyclisch-aromatische Reste eines 5- oder 6-gliedrigen heterocyclischen Ringsystems mit 1 oder 2 Heteroatomen aus der Reihe O, N, S, an das ein Benzolring ankondensiert sein kann, substituiert sein können, Phenyl, Naphthyl, Phenyl-$C_1$–$C_4$-alkyl und Naphthyl-$C_1$–$C_4$-alkyl, die durch Cl, Br, F, $C_1$–$C_{18}$-Alkyl, $C_1$–$C_{18}$-Alkoxy substituiert sein können, den Rest eines 5- oder 6-gliedrigen heterocyclischen Ringes mit 1, 2 oder 3 Heteroatomen aus der Reihe O, N, S, an den ein Benzolring ankondensiert sein kann, bezeichnen oder $R_7$ und $R_8$ bilden in der Gruppe–$CON(R_7)(R_8)$ zusammen unter Einschluß des N-Atoms einen 5- oder 6-gliedrigen heterocyclischen Ring,

$R_9$ $C_1$–$C_{18}$-Alkyl, Cyclopentyl, Cyclohexyl, Phenyl-$C_1$–$C_4$-alkyl, Naphthyl-$C_1$–$C_4$-alkyl, Phenyl, Naphthyl, oder den Rest eines 5- oder 6-gliedrigen heterocyclischen Ringes mit 1, 2 oder 3 Heteroatomen aus der Reihe O, N, S, an den ein Benzolring ankondensiert sein kann, wobei die für $R_9$ genannten Reste wie die entsprechenden Reste $R_7$, $R_8$ substituiert sein können,

$R_{10}$ Wasserstoff, $-COR_9$ oder $R_7$,

$R_{11}$ den Rest einer Kupplungskomponente aus der Benzol-, Naphthalin-, Acetessigsäureanhydrid-, Pyrazol- oder Pyridonreihe oder einen gegebenenfalls durch Cl, Br, F, $C_1$–$C_{18}$-Alkyl und $C_1$–$C_{18}$-Alkoxy substituierten Phenylrest,
$R_{12}$ Wasserstoff, $C_1$–$C_8$-Alkyl, gegebenenfalls durch F, Cl, Br, $-NO_2$, $-CN$, $-CF_3$, $C_1$–$C_{18}$-Alkyl, $C_1$–$C_{18}$-Alkoxy, Phenyl, Phenoxy, $C_1$–$C_6$-Alkoxycarbonyl und Phenoxycarbonyl substituiertes Phenyl oder Naphthyl bezeichnen,
$R_{13}$ für Wasserstoff steht oder die Bedeutungen von $R_3$, $R_4$, ausgenommen den Rest der Formel Ia, annehmen kann,
$R_{14}$ für ein zweiwertiges gesättigtes oder ungesättigtes aliphatisches Brückenglied mit 1–8 C-Atomen, einen Phenylen- oder Xylylenrest oder einen Rest der Formeln

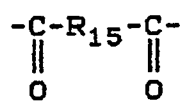 und 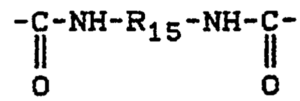

steht, wobei $R_{15}$ einen $C_1$–$C_{10}$-Alkylen-, einen Phenylen-, Toluylen- oder Naphthylenrest bezeichnet, und
A für einen Rest der Formel

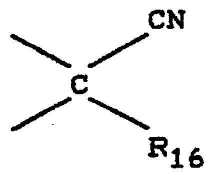

worin
$R_{16}$ Cyan, $C_1$–$C_6$-Alkoxy-$C_1$–$C_6$-alkoxycarbonyl, gegebenenfalls durch $C_1$–$C_6$-Alkyl, Benzyl, Naphthyl oder Phenyl substituiertes Carbamoyl, wobei Phenyl, Benzyl, Naphthyl durch Chlor, Brom, $C_1$–$C_6$-Alkyl, $C_1$–$C_6$-Alkoxy, Nitro, Trifluormethyl, $C_1$–$C_6$-Alkylcarbonyl, Cyan, $C_1$–$C_6$-Alkylamino, Benzoylamino, Phthalimidyl, Carbamoyl, substituiert sein können, $C_1$–$C_6$-Alkylcarbonyl; Benzoyl, $C_1$–$C_6$-Alkoxycarbonyl, Benzyloxycarbonyl, Phenoxycarbonyl, wobei Benzoyl, Benzyl und Phenoxy durch Halogen, Nitro, $C_1$–$C_6$-Alkyl, $C_1$–$C_6$-Alkylcarbonylamino, Phthalimidyl, substituiert sein können oder gegebenenfalls durch Halogen, Nitro, Cyan, Trifluormethyl substituiertes Phenyl bezeichnet oder einen Rest der Formel

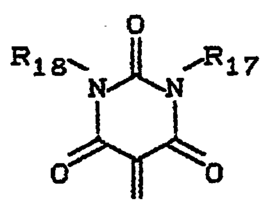

in der
$R_{17}$, $R_{18}$ Wasserstoff, $C_1$–$C_6$-Alkyl, gegebenenfalls durch Halogen, $C_1$–$C_6$-Alkyl, $C_1$–$C_6$-Alkoxy oder Nitro substituiertes Phenyl, α-Naphthyl und β-Naphthyl
bezeichnen, steht.

Die heterocyclisch-aromatischen Reste $R_1$, $R_2$ enthalten insbesondere 1 oder 2 fünf-, sechs- oder siebengliedrige Ringe, von denen mindestens einer bevorzugt 1 oder 2 Heteroatome aus der Reihe O, N, S enthält.

Als heterocyclisch-aromatische Reste seien beispielhaft genannt:

Pyridyl, Pyrimidyl, Pyrazinyl, Triazinyl, Furanyl, Pyrrolyl, Thienyl, Chinolyl, Cumarinyl, Benzofuranyl, Benzoimidazolyl, Benzoxazolyl, Dibenzofuranyl, Benzothienyl, Dibenzothienyl, Indolyl, Carbazolyl, Pyrazolyl, Imidazolyl, Oxazolyl, Isooxazolyl, Thiazolyl, Indazolyl, Benzthiazolyl, Pyridazinyl, Cinnolyl, Chinazolyl, Chinoxalyl, Phthalazinyl, Phthalazindionyl, Phthalimidyl, Chromonyl, Naphtholactamyl, Benzopyridonyl, ortho-Sulfobenzimidyl, Maleinimidyl, Naphtharidinyl, Benzimidazolonyl, Benzoxazolonyl, Benzthiazolonyl, Benzthiazolinyl, Chinazolonyl, Pyrimidonyl, Chinoxalonyl, Phthalazonyl, Dioxapyrimidinyl, Pyridonyl, Isochinolonyl, Isochinolinyl, Isothiazolyl, Benzisoxazolyl, Benzisothiazolyl, Indazolonyl, Acridinyl, Acridonyl, Chinazolindionyl, Chinoxalindionyl, Benzoxazindionyl, Benzoxazinonyl und Naphthalimidyl.

$R_1$, $R_2$ stehen bevorzugt für

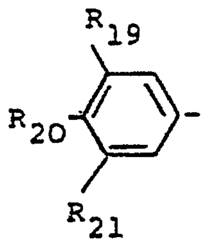

wobei

$R_{19}$, $R_{20}$, $R_{21}$ Wasserstoff, Halogen wie Cl, Br, F, –CN, $-R_5$, $-OR_6$, $-SR_6$, $-N(R_7)(R_8)$, $-COOR_9$, $-N(R_{10})COR_9$, $-N(R_6)COOR_9$, $-N(R_6)CON(R_7)(R_8)$, $-NHSO_2R_9$, $-SO_2R_9$, $-SOR_9$, $-SO_2OR_9$, $-COR_7)(R_8)$, $-SO_2N(R_7)(R_8)$, $-N{=}N{-}R_{11}$, $-OCOR_9$ und $-OCONHR_9$ mit den o.a. Bedeutungen für $R_5$, $R_6$, $R_7$, $R_8$, $R_9$, $R_{10}$ und $R_{11}$

bezeichnen.

Besonders bevorzugt sind Verbindungen mit $R_{19}$, $R_{20}$=H und $R_{21}$≠H sowie mit $R_{19}$, $R_{21}$=H und $R_{20}$≠H.

Besonders bevorzugt stehen $R_1$, $R_2$ für einen Rest der Formel

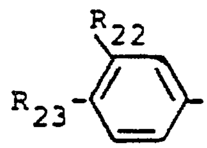

in der

$R_{22}$, $R_{23}$ Wasserstoff, Halogen wie Chlor, Brom, Fluor, $CF_3$, $C_1$–$C_4$-Alkyl, insbesondere Methyl und Ethyl, –CN, $C_1$–$C_4$-Alkoxy, insbesondere Methoxy und Ethoxy, Mono- oder Di-$C_1$–$C_4$-Alkylamino, insbesondere Di-methylamino, gegebenenfalls durch Chlor substituiertes Benzoyl oder Benzoylamino, Naphthyl oder Reste der Formeln

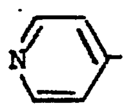 , 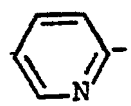 , 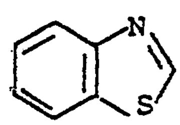 ,

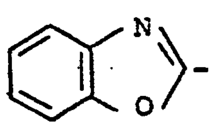 , 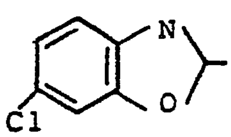

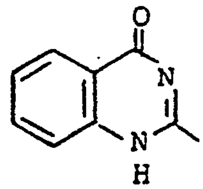 , 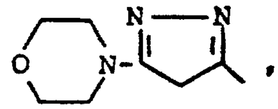 ,

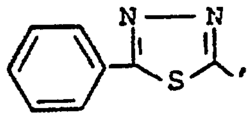 , 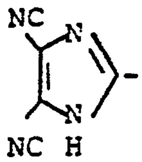 , 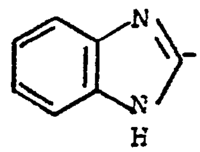

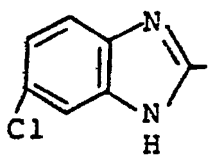

bezeichnen.

$R_3$, $R_4$ stehen besonders bevorzugt für Wasserstoff.

Von besonderem Interesse ist der Farbstoff der Formel I, bei dem $R_1$, $R_2$ 4-Chlorphenyl und $R_3$, $R_4$ Wasserstoff bedeuten.

Zur Herstellung der Verbindungen der Formel I setzt man Verbindungen, die in einer ihrer tautomeren Formen der Formel

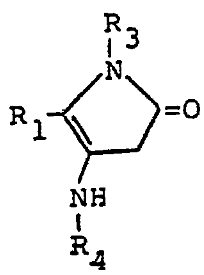

II

entsprechen, mit Verbindungen der Formel

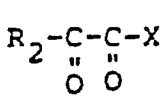

III

um und setzt aus dem entstandenen Reaktionsprodukt durch Hydrolyse die Verbindungen der Formel I frei.

In den Formeln II und III haben $R_1$, $R_2$, $R_3$ und $R_4$ die zu Formel I angegebenen Bedeutungen; X bezeichnet Halogen wie Chlor und Brom oder Alkoxy, Cycloalkoxy, Aryloxy, Aralkoxy, insbesondere $C_1$–$C_4$-Alkoxy.

Die praktische Durchführung der Umsetzung von Verbindungen der Formel II mit den Ketoestern der Formel III erfolgt ähnlich zu der aus der EP-A3 94 911 bekannten Umsetzung von Nitrilen mit Bernsteinsäurediester zu 1,4-Diketo-pyrrolo-[3,4-c]-pyrrolen.

Man führt die Umsetzung des Ketoesters der Formel III mit der Verbindung der Formel II vorzugsweise in einem Lösungsmittel durch.

Als Lösungsmittel eignen sich beispielsweise primäre, sekundäre oder tertiäre Alkohole mit 1 bis 10 C-Atomen, wie Methanol, Ethanol, n-Propanol, Isopropanol, n-Butanol, sek.-Butanol, tert.-Butanol, n-Pentanol, 2-Methyl-2-butanol, 2-Methyl-2-pentanol, 3-Methyl-3-pentanol, 2-Methyl-2-hexanol, 3-Ethyl-3-pentanol, 2,4,4-Trimethyl-2-pentanol oder Glykole, wie Ethylenglykol oder Diethylenglykol, ferner Ether, wie Tetrahydrofuran oder Dioxan, oder Glykolether, wie Ethylenglykol-methylether, Ethylenglykol-ethylether, Diethylenglykol-monomethylether oder Diethylenglykol-monoethylether, ferner dipolaraprotische Lösungsmittel, wie Acetonitril, Benzonitril, Dimethylformamid, N,N-Dimethylacetamid, Nitrobenzol, N-Methylpyrrolidon, aliphatische oder aromatische Kohlenwasserstoffe, wie Benzol oder durch Alkyl, Alkoxy oder Halogen substituiertes Benzol, wie Toluol, Xylol, Anisol oder Chlorbenzol oder aromatische N-Heterocyclen, wie Pyridin, Picolin oder Chinolin.

Zweckmäßigerweise verwendet man 5–20 Gew.-Tle. Lösungsmittel auf 1 Gew.-Tl. der Reaktionsteilnehmer.

Im erfindungsgemäßen Verfahren verwendet man bevorzugt einen Alkohol als Lösungsmittel, insbesondere einen sekundären oder tertiären Alkohol. Bevorzugte tertiäre Alkohole sind tert.-Butanol und tert.-Amylalkohol. Dabei sind auch Gemische dieser bevorzugten Lösungsmittel mit aromatischen Kohlenwasserstoffen, wie Toluol oder Xylol, oder mit durch Halogen substituiertem Benzol, wie Chlorbenzol, von großem Interesse.

Das erfindungsgemäße Verfahren wird in Gegenwart einer starken Base durchgeführt. Geeignete Basen sind insbesondere die Alkalimetalle selbst, wie Lithium, Natrium oder Kalium, oder Alkaliamide, wie Lithium-, Natrium- oder Kaliumamid oder Alkalihydride, wie Lithium-, Natrium- oder Kaliumhydrid, oder Erdalkali- oder Alkalialkoholate, die sich insbesondere von primären, sekundären oder tertiären aliphatischen Alkoholen mit 1 bis 10 C-Atomen ableiten, wie z.B. Lithium-, Natrium- oder Kaliummethylat, e-thylat, -n-propylat, -isopropylat, -n-butylat, -sek.-butylat, -tert.-butylat, -2-methyl-2-butylat, -2-methyl-2-pentylat, -3-methyl-3-pentylat, -3-ethyl-3-pentylat. Man kann aber auch ein Gemisch der genannten Basen verwenden.

Im erfindungsgemäßen Verfahren verwendet man als starke Base bevorzugt Alkalialkoholate, wobei Alkali insbesondere Natrium oder Kalium bedeutet, und das Alkoholat sich bevorzugt von einem sekundären oder tertiären Alkohol ableitet. Besonders bevorzugte starke Basen sind daher z.B. Natrium- oder Kalium-isopropylat, -sek.-butylat, -tert.-butylat und -tert.-amylat. Dabei können die Alkalialkoholate auch in situ hergestellt werden, indem man den entsprechenden Alkohol mit dem Alkalimetall, Alkalihydrid oder Alkaliamid umsetzt.

Im erfindungsgemäßen Verfahren kann man die starke Base in einer Menge von insbesondere 0,1 bis 10 Mol, bevorzugt 1,9 bis 4,0 Mol, bezogen auf 1 Mol der Verbindung der Formel II einsetzen. Obwohl grundsätzlich stöchiometrische Mengen an Base genügen, beeinflußt in vielen Fällen ein Basenüberschuß die Ausbeute günstig.

Das erfindungsgemäße Verfahren wird insbesondere bei einer Temperatur von 60 bis 140°C, vorzugsweise 70 bis 120°C, durchgeführt.

Zur Hydrolyse des Kondensationsproduktes kann man Wasser, einen Alkohol mit 1 bis 4 C-Atomen, wie Methanol oder Ethanol, vorzugsweise aber eine Säure, verwenden. Als Säuren kommen z.B. aliphatische oder aromatische Carbon- oder Sulfonsäuren in Betracht, wie beispielsweise Ameisensäure, Essigsäure, Propionsäure, Oxalsäure, Benzoesäure oder Benzolsulfonsäure. Weiterhin kommen als Säuren auch Mineralsäuren in Betracht, wie Salzsäure, Schwefelsäure oder Phosphorsäure. Vorzugsweise benutzt man zur Hydrolyse eine organische Säure, insbesondere eine aliphatische Carbonsäure, wie Essigsäure.

Bei der Hydrolyse fällt die Verbindung der Formel I aus und kann durch Abfiltrieren isoliert werden.

Zur Umsetzung der Reaktanden ist es grundsätzlich möglich, bei tieferer Temperatur alle Komponenten vorzulegen und dann das Gemisch in dem Bereich der Reaktionstemperatur zu erwärmen, oder in beliebiger Reihenfolge die einzelnen Komponenten im Bereich der Reaktionstemperatur zueinander zuzugeben. Eine bevorzugte Ausführungsform, die sich in der Regel besonders günstig auf die Ausbeute auswirkt, besteht darin, daß man die Verbindung der Formel II zusammen mit der Base vorlegt und den α-Ketoester im Bereich der Reaktionstemperatur zudosiert. Eine weitere Möglichkeit besteht darin, daß man eine Mischung der Reaktanden gleichzeitig zur vorgelegten Base zudosiert. Es ist möglich, das erfindungsgemäße Verfahren nicht nur batchweise, sondern auch kontinuierlich durchzuführen.

Verwendet man als Lösungsmittel einen Alkohol und als Base ein Alkoholat, so kann es vorteilhaft sein, einen Alkohol und ein Alkoholat mit gleichen Alkylteilen zu wählen. Ebenso vorteilhaft kann es sein, wenn zudem noch der α-Ketoester ebenso solche Alkylgruppen enthält.

Wird zur Herstellung der Verbindungen der Formel I an Stelle des α-Ketoesters der Formel III das Säurehalogenid der Formel III (X = Br, bevorzugt Cl) eingesetzt, so wird bevorzugt in aprotischen Lösungsmitteln wie Dioxan, Tetrahydrofuran, Pyridin, Diglykolether wie Diethylenglykol-monomethylether oder Diethylenglykol-monoethylether, Dimethylformamid, Benzol, Toluol, Xylol o.ä. gearbeitet, wobei mindestens ein weiteres Äquivalent der Base eingesetzt werden muß.

Die Verbindungen der Formel III sind bekannt oder in Analogie zu literaturbekannten Verfahren herstellbar.

Zur Herstellung der Verbindungen der Formel II setzt man Verbindungen der Formel

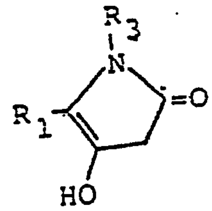

IV

die als "Tetramsäuren" aus zahlreichen Literaturstellen bekannt sind (z.B. aus Heterocycles 13 (1979) 477–495 und dort zitierte Literaturstellen) mit Aminen der Formel

$$H_2N–R_4$$

in an sich bekannter Weise (vgl. die bekannten Umsetzungen von β-Dicarbonylverbindungen mit Aminen zu Enaminen) um.

Die Verbindungen der Formel I, bei denen $R_3$, $R_4$ für

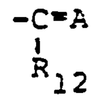

stehen, können z.B. hergestellt werden, indem man Verbindungen der Formel

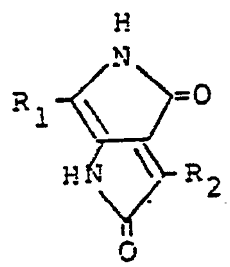

V

mit Verbindungen der Formel

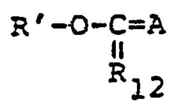

VI

wobei R′ vorzugsweise $C_1$–$C_4$-Alkyl bezeichnet, in an sich bekannter Weise umsetzt.

Die gelben bis blauen Verbindungen der Formel I finden vorzugsweise als Pigmente Verwendung, können je nach ihrer Struktur und der Art der zu färbenden Polymeren jedoch auch als polymerlösliche Farbstoffe für z.B. Polystyrol, Polyamide, ABS, vor allem für lineare Polyester, insbesondere Polyethylenterephthalate eingesetzt werden.

Die Verbindungen der Formel I fallen in einer für die Pigmentanwendung geeigneten Form an oder können durch an sich bekannte Nachbehandlungsverfahren in die geeignete Form überführt werden, z.B. durch Lösen oder Quellen in starken anorganischen Säuren wie Schwefelsäure und Austragen auf Eis. Die Feinverteilung kann auch durch Mahlen mit oder ohne Mahlhilfsstoffen wie anorganischen Salzen oder Sand, gegebenenfalls in Anwesenheit von Lösungsmitteln wie Toluol, Xylol, Dichlorbenzol oder N-Methyl-pyrrolidon erzielt werden. Farbstärke und Transparenz des Pigmentes können durch Variation der Nachbehandlung beeinflußt werden.

Die Verbindungen der Formel I eignen sich aufgrund ihrer Licht- und Migrationsechtheit für die verschiedensten Pigmentapplikationen. So können sie zur Herstellung von sehr echt pigmentierten Systemen, wie Mischung mit anderen Stoffen, Zubereitungen, Anstrichmitteln, Druckfarben, gefärbtem Papier und gefärbten makromolekularen Stoffen verwendet werden. Unter Mischung mit anderen Stoffen können z.B. solche mit anorganischen Weißpigmenten wie Titandioxid (Rutil) oder mit Zement verstanden werden. Zubereitungen sind z.B. Flushpasten mit organischen Flüssigkeiten oder Teige und Feinteige mit Wasser, Dispergiermitteln und gegebenenfalls Konservierungsmitteln. Die Bezeichnung Anstrichmittel steht z.B. für physikalisch oder oxidativ trocknende Lacke, Einbrennlacke, Reaktionslacke, Zweikomponentenlacke, Dispersionsfarben für wetterfeste Überzüge und Leimfarben. Unter Druckfarben sind solche für den Papier-, Textil- und Blechdruck zu verstehen. Die makromolekularen Stoffe können natürlichen Ursprungs sein wie Kautschuk, durch chemische Modifikation erhalten werden wie Acetylcellulose, Cellulosebutyrat oder Viskose oder synthetisch erzeugt werden wie Polymerisate, Polyadditionsprodukte und Polykondensate. Genannt seien plastische Massen wie Polyvinylchlorid, Polyvinylacetat, Polyvinylpropionat, Polyolefine, z.B. Polyethylen oder Polyamide, Superpolyamide, Polymerisate und Mischpolymerisate aus Acrylester, Methacrylestern, Acrylnitril, Acrylamid, Butadien, Styrol sowie Polyurethane und Polycarbonate. Die mit den beanspruchten Produkten pigmentierten Stoffe können in beliebiger Form vorliegen.

Die Pigmente der Formel I sind weiterhin ausgezeichnet wasserecht, ölecht, säureecht, kalkecht, alkaliecht, lösungsmittelecht, überlackierecht, überspritzecht, sublimierecht, hitzebeständig, vulkanisierbeständig, sehr ergiebig und in plastischen Massen gut verteilbar.

Die folgenden Beispiele erläutern die Erfindung.

Beispiel 1 (Herstellung des Ausgangsprodukts)

18 g 5-Phenyl-tetramsäure werden in 200 ml n-Butanol verrührt. Die Lösung wird unter gleichzeitigem Einleiten von gasförmigem Ammoniak langsam zum Rückfluß erhitzt. Es wird 1 Stunde am Rückfluß gekocht, wobei ständig ein schwacher Ammoniakstrom durch das Reaktionsgemisch geleitet wird. Anschließend wird das Reaktionsgemisch am Rotationsverdampfer im Vakuum zur Trockene eingeengt. Man erhält 17 g Substanz, die in einer ihrer tautomeren Formen der Formel

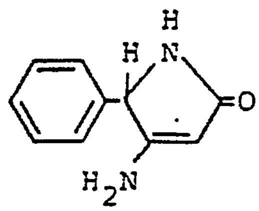

entspricht.

Beispiel 2

9 g der in Beispiel 1 hergestellten Verbindung werden in 200 ml tert.-Butanol mit 18 g Kalium-tert.-butylat und 10 ml Phenylglyoxylsäuremethylester 5 Stunden am Rückfluß gekocht. Man läßt auf 50°C abkühlen, versetzt die Suspension mit 300 ml Methanol und 25 ml Eisessig. Die ausgefallenen Kristalle werden isoliert, gut mit Methanol und Wasser gewaschen und getrocknet. Man erhält 6 g einer blauvioletten Substanz, die in einer ihrer tautomeren Formen der Formel

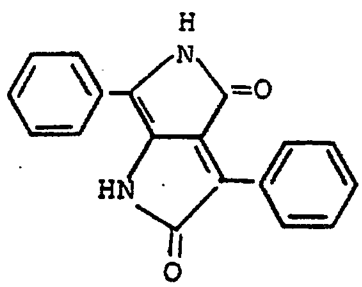

Massenspektroskopie: MS: $M^+$: 288 als Basepeak
UV (DMF): $\lambda_{max} = 554$ nm
entspricht, und die in PVC eingearbeitet eine violette Färbung ergibt.

Beispiel 3

11 g 2-Hydroxy-5-(4′-chlorphenyl)-4-amino-pyrrol werden in 200 ml tert.-Butanol mit 18 g Kalium-tert.-butylat zum Rückfluß erhitzt. Im Verlaufe von 2 Stunden werden 15 ml Phenylglyoxylsäuremethylester zugetropft. Nach 3-stündigem Rückflußkochen wird eine Mischung aus 200 ml Methanol und 20 ml Eisessig zugetropft. Die ausgefallenen Kristalle werden isoliert, mit Methanol und Wasser gewaschen und getrocknet. Man erhält 12 g Substanz der Formel

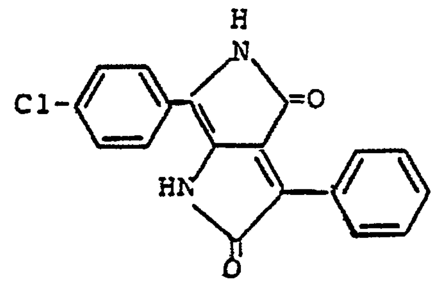

Beispiel 4

9 g der in Beispiel 1 hergestellten Verbindung werden nach den Angaben des Beispiels 2 mit 15 g p-Chlorphenylglyoxylsäureethylester umgesetzt. Man erhält 11 g Substanz, die mit der nach Beispiel 3 hergestellten identisch ist.

Beispiel 5

11 g 2-Hydroxy-5-(4′-chlorphenyl)-4-amino-pyrrol werden in 200 ml tert.-Amylalkohol mit 18 g Kalium-tert.-butylat und 15 g p-Chlorphenylglyoxylsäuremethylester 4 Stunden auf 100°C erwärmt, wobei man das entstehende Methanol abdestilliert. Nach Hydrolyse mit Methanol-Eisessig, Isolierung und Trocknung erhält man 15 g Pigment der Formel

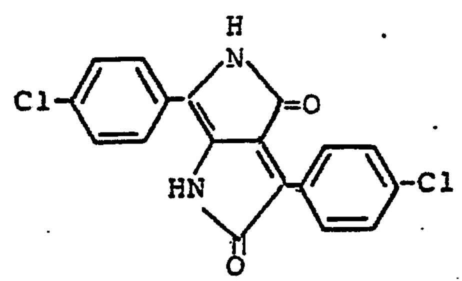

welches in PVC eingearbeitet eine blauviolette Färbung ergibt.

Beispiel 6

7 g Natrium werden in 200 ml wasserfreiem Ethanol gelöst. Man fügt 9 g der in Beispiel 1 hergestellten Verbindung zu und tropft bei 78°C im Verlaufe von 1 Stunde 15 ml Phenylglyoxylsäureethylester zu. Nach beendeter Zudosierung wird das Reaktionsgemisch noch 3 Stunden am Rückfluß gekocht und dann wie in Beispiel 2 aufgearbeitet. Man erhält 12 g Pigment, welches mit dem nach Beispiel 2 hergestellten identisch ist.

Beispiel 7–53

Analog Beispiel 3 erhält man weitere Pigmente der Formel

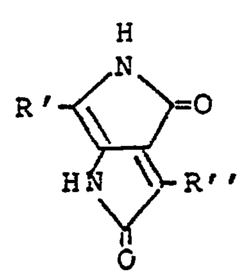

worin R′ und R″ die in der folgenden Tabelle aufgeführten Bedeutungen haben.

| Beispiel | R' | R'' |
|---|---|---|
| 7 | 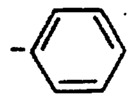 | 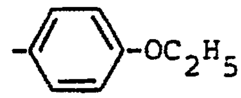 |
| 8 | 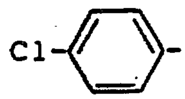 | 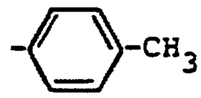 |
| 9 | 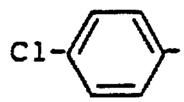 | 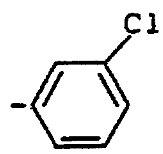 |
| 10 | 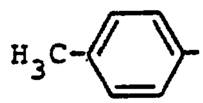 | 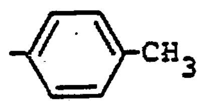 |
| 11 | 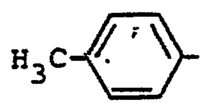 | 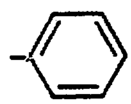 |
| 12 | 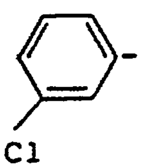 | 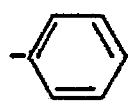 |
| 13 | 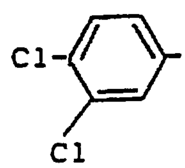 | 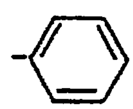 |
| 14 | 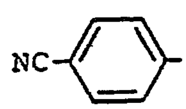 | 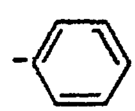 |

| Beispiel | R' | R'' |
|---|---|---|
| 15 | 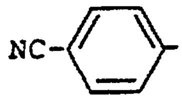 | 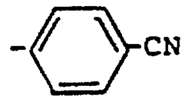 |
| 16 | 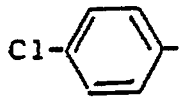 | 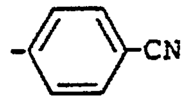 |
| 17 | 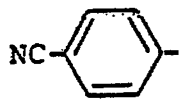 | 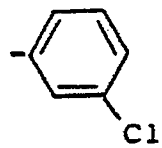 |
| 18 | 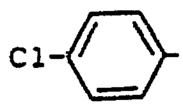 | 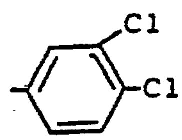 |
| 19 | 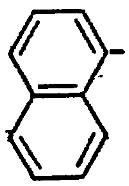 | 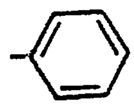 |
| 20 | 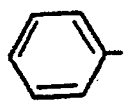 | 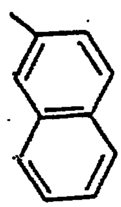 |
| 21 | 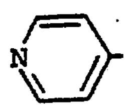 | 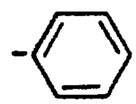 |

| Beispiel | R' | R'' |
|---|---|---|
| 22 | 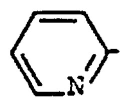 | 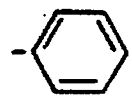 |
| 23 | 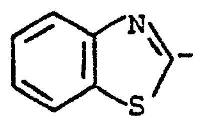 | 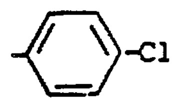 |
| 24 | 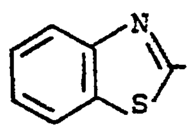 | 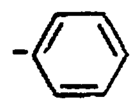 |
| 25 | 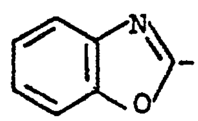 | 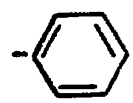 |
| 26 | 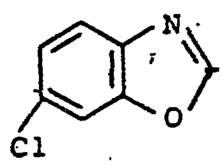 | 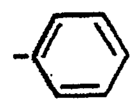 |
| 27 | 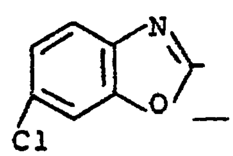 | 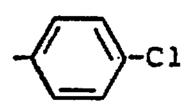 |
| 28 | 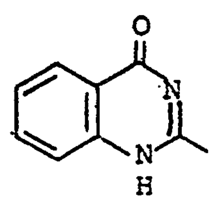 | 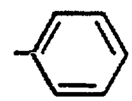 |

| Beispiel | R' | R'' |
|---|---|---|
| 29 | 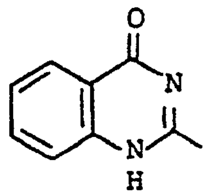 | 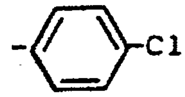 |
| 30 | 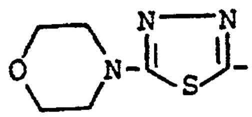 | 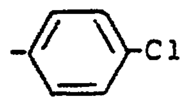 |
| 31 | 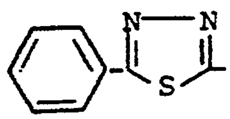 | 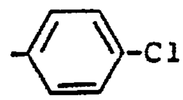 |
| 32 | 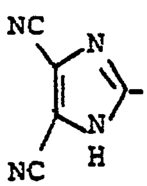 | 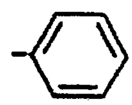 |
| 33 | 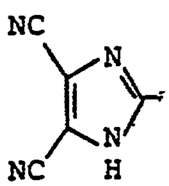 | 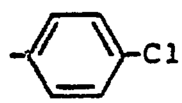 |
| 34 | 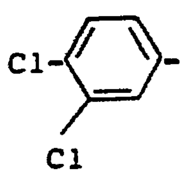 | 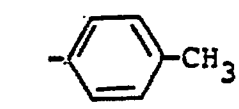 |
| 35 | 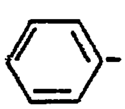 | 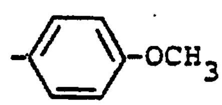 |

| Beispiel | R′ | R′′ |
|---|---|---|
| 36 | 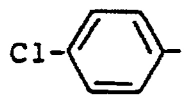 | 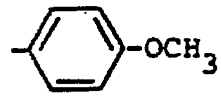 |
| 37 | 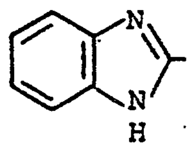 | 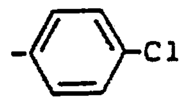 |
| 38 | 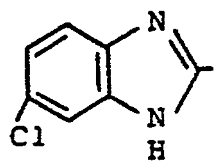 | 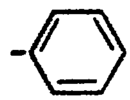 |
| 39 | 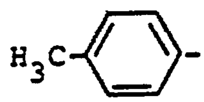 | 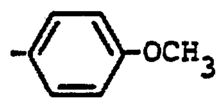 |
| 40 | 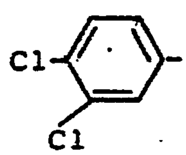 | 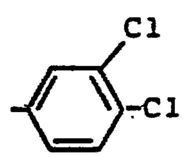 |
| 41 | 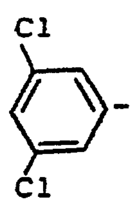 | 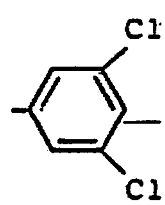 |
| 42 | 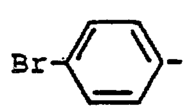 | 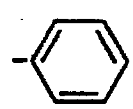 |

| Beispiel | R' | R'' |
|---|---|---|
| 43 | 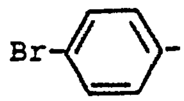 | 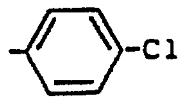 |
| 44 | 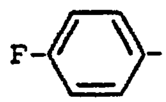 | 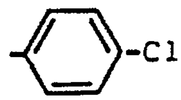 |
| 45 | 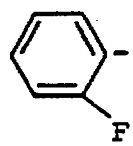 | 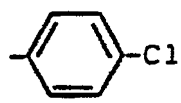 |
| 46 | 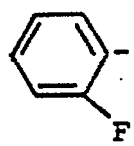 | 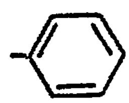 |
| 47 | 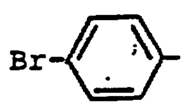 | 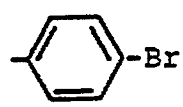 |
| 48 | 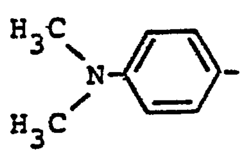 | 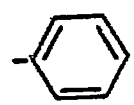 |
| 49 | 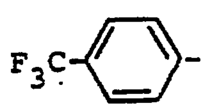 | 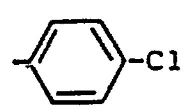 |

| Beispiel | R' | R'' |
|---|---|---|
| 50 | 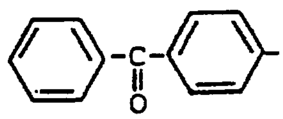 | 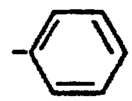 |
| 51 | 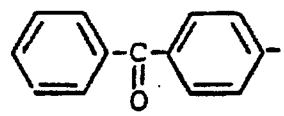 | 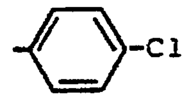 |
| 52 | 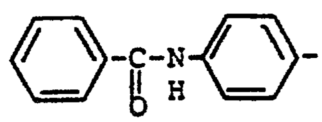 | 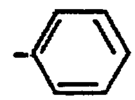 |
| 53 | 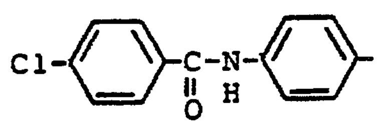 | 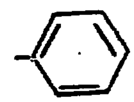 |

Beispiel 54 (Anwendungsbeispiel)

4 g feingemahlenes Pigment gemäß Beispiel 2 werden in 92 g eines Einbrennlackes folgender Zusammensetzung dispergiert:
33% Alkydharz
15% Melaminharz
5% Glykolmonomethylether
34% Xylol
13% Butanol

Als Alkydharze kommen Produkte auf Basis synthetischer und pflanzlicher Fettsäuren wie Kokosöl, Rizinusöl, Rizinenöl, Leinöl u.a. in Frage. Anstelle von Melaminharzen können Harnstoffharze verwendet werden.

Nach erfolgter Dispergierung wird der pigmentierte Lack auf Papier-, Glas-, Kunststoff- oder Metall-Folien aufgetragen und 30 Minuten bei 130°C eingebrannt. Die Lackierungen besitzen sehr gute Licht- und Wetterbeständigkeit sowie gute Überlackierechtheit.

Der Einbrennlack, hergestellt gemäß Beispiel 54, wird auf weißes Papier aufgestrichen und bei 130°C eingebrannt.

Beispiel 55

Eine Mischung aus 65 g Polyvinylchlorid, 35 g Diisooctylphthalat, 2 g Dibutylzinnmercaptid, 0,5 g Titandioxid und 0,5 g des Pigmentes von Beispiel 5 wird auf einem Mischwalzwerk bei 165°C eingefärbt. Man erhält eine intensiv blauviolett gefärbte Masse, die zur Herstellung von Folien oder Formkörpern dienen kann. Die Färbung zeichnet sich durch hervorragende Licht- und sehr gute Weichmacherechtheit aus.

Beispiel 56

0,2 g Pigment nach Beispiel 2 werden mit 100 g Polyethylen-, Polypropylen oder Polystyrolgranulat gemischt. Die Mischung kann entweder bei 220 bis 280°C direkt in einer Spritzgußmaschine verspritzt, oder in einer Strangpresse zu gefärbten Stäben bzw. auf dem Mischwalzwerk zu gefärbten Fellen verarbeitet werden. Die Stäbe bzw. Felle werden gegebenenfalls granuliert und in einer Spritzgußmaschine verspritzt.

Die violetten Formlinge besitzen sehr gute Licht- und Migrationsechtheit. In ähnlicher Weise können bei 280–300°C, gegebenenfalls unter Stickstoffatmosphäre, synthetische Polyamide aus Caprolactam oder Adipinsäure und Hexamethylendiamin oder die Kondensate aus Terephthalsäure und Ethylenglykol gefärbt werden.

Beispiel 57

1 g Pigment nach Beispiel 5, 10 g Titandioxid (Rutiltyp) und 100 g eines in Pulverform vorliegenden Mischpolymerisates auf Basis von Acrylnitril-Butadien-Styrol werden gemischt und auf einem Walzwerk bei 140–180°C eingefärbt. Man erhält ein orangefarbenes Fell, das granuliert und in einer Spritzgußmaschine bei 200–250°C verspritzt wird. Man erhält blauviolette Formlinge sehr guter Licht- und Migrationsechtheit sowie ausgezeichneter Hitzebeständigkeit.

Auf ähnliche Weise, jedoch bei Temperaturen von 180–220°C und ohne Zusatz von Titandioxid werden Kunststoffe auf Basis von Celluloseacetat, Cellulosebutyrat und deren Gemische mit ähnlichen Echtheiten gefärbt.

Beispiel 58

0,2 g Pigment nach Beispiel 5 werden in feinverteilter Form mit 100 g eines Kunststoffes auf Polycarbonat-Basis in einem Extruder oder in einer Knetschnecke bei 250–280°C gemischt und zu Granulat verarbeitet. Man erhält ein blauviolettes transparentes Granulat hervorragender Lichtechtheit und Hitzebeständigkeit.

**Patentansprüche**

1. 2,4-Dioxopyrrolo[2,3-c]pyrrole, die in einer ihrer tautomeren Formen der Formel

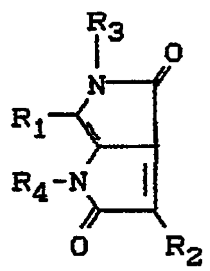

(I)

entsprechen, in der

$R_1$, $R_2$ Phenyl, Diphenylyl, Naphthyl oder einen heterocyclisch-aromatischen Rest, der 1, 2, 3 oder 4 fünf-, sechs- oder sieben-gliedrige Ringe enthält, von denen mindestens einer 1, 2 oder 3 Heteroatome aus der Reihe N, O, S enthält, bezeichnen, wobei die für $R_1$, $R_2$ genannten Reste durch Halogen, –CN, $-R_5$, $OR_6$, $-SR_6$, $-N(R_7)(R_8)$, $-COOR_9$, $-N(R_{10})COR_9$, $-N(R_6)COOR_9$, $N(R_6)CON(R_7)(R_8)$, $-NH\ SO_2R_9$, $-SO_2R_9$, $-SOR_9$, $-SO_2OR_9$, $-CON(R_7)(R_8)$, $-SO_2N(R_7)(R_8)$, $-N{=}N-R_{11}$, $-OCOR_9$ und $-OCONHR_9$ substituiert sein können,

$R_3$, $R_4$ für Wasserstoff, $C_2$–$C_{18}$-Alkenyl und $C_2$–$C_{18}$-Alkinyl, die durch Halogen, –CN, $-OCOR_9$, $-OR_7$, $-COOR_9$, $-SR_7$, $-CON(R_7)(R_8)$, $-OCONHR_9$ substituiert sein können, Epoxyethyl, Phenyl und Naphthyl, die durch F, Cl, Br, $-NO_2$, –CN, $-CF_3$, $C_1$–$C_{18}$-Alkyl, $C_1$–$C_{18}$-Alkoxy, Phenyl, Phenoxy, $C_1$–$C_6$-Alkoxy-carbonyl und Phenoxycarbonyl substituiert sein können, $-R_5$, $-COR_6$,

$-CON(R_7)(R_8)$, 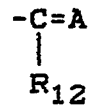 oder einen Rest

der Formel

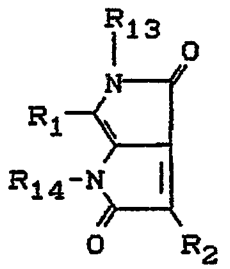

(Ia)

stehen und

$R_5$ $C_1$–$C_{18}$-Alkyl oder $C_3$–$C_7$-Cycloalkyl, die durch Halogen, –CN, $-OCOR_9$, $-OR_7$, $-COOR_9$, $-SR_7$, $-CON(R_7)(R_8)$ und $-OCONHR_9$ substituiert sein können,

$R_6$, $R_7$, $R_8$ Wasserstoff, $C_1$–$C_{18}$-Alkyl, Cyclopentyl, Cyclohexyl, die durch Cl, Br, F, –CN, –OH, Mono-$C_1$–$C_4$-alkyl-amino, Di-$C_1$–$C_4$-alkylamino, gegebenenfalls durch Cl, Br, F, $C_1$–$C_6$-Alkyl und $C_1$–$C_6$-Alkoxy substituiertes Phenyl oder Naphthyl, oder heterocyclisch-aromatische Reste eines 5- oder 6-gliedrigen heterocyclischen Ringsystems mit 1 oder 2 Heteroatomen aus der Reihe O, N, S an das ein Benzolring ankondensiert sein kann, substituiert sein können, Phenyl, Naphthyl, Phenyl-$C_1$–$C_4$-alkyl und Naphthyl-$C_1$–$C_4$-alkyl, die durch Cl, Br, F, $C_1$–$C_{18}$-Alkyl, $C_1$–$C_{18}$-Alkoxy substituiert sein können, den Rest eines 5- oder 6-gliedrigen heterocyclischen Ringes mit 1, 2 oder 3 Heteroatomen aus der Reihe O, N, S an den ein Benzolring ankondensiert sein kann, bezeichnen oder $R_7$ und $R_8$ bilden in der Gruppe $-CON(R_7)(R_8)$ zusammen unter Einschluß des N-Atoms einen 5- oder 6-gliedrigen heterocyclischen Ring,

$R_9$ $C_1$–$C_{18}$-Alkyl, Cyclopentyl, Cyclohexyl, Phenyl-$C_1$–$C_4$-alkyl, Naphthyl-$C_1$–$C_4$-alkyl, Phenyl, Naphthyl, oder den Rest eines 5- oder 6-gliedrigen heterocyclischen Ringes mit 1, 2 oder 3 Heteroatomen aus der Reihe O, N, S, an den ein Benzolring ankondensiert sein kann, wobei die für $R_9$ genannten Reste wie die entsprechenden Reste $R_7$, $R_8$ substituiert sein können,

$R_{10}$ Wasserstoff, $-COR_9$ oder $R_7$,

$R_{11}$ den Rest einer Kupplungskomponente aus der Benzol-, Naphthalin-, Acetessigsäureanhydrid-, Pyrazol- oder Pyridonreihe oder einen gegebenenfalls durch Cl, Br, F, $C_1$–$C_{18}$-Alkyl und $C_1$–$C_{18}$-Alkoxy substituierten Phenylrest,

$R_{12}$ Wasserstoff, $C_1$–$C_8$-Alkyl, gegebenenfalls durch F, Cl, Br, $-NO_2$, –CN, $-CF_3$, $C_1$–$C_{18}$-Alkyl, $C_1$–$C_{18}$-Alkoxy, Phenyl, Phenoxy, $C_1$–$C_6$-Alkoxycarbonyl und Phenoxycarbonyl substituiertes Phenyl oder Naphthyl bezeichnen,

$R_{13}$ für Wasserstoff steht oder die Bedeutungen von $R_3$, $R_4$, ausgenommen den Rest der Formel Ia, annehmen kann,

$R_{14}$ für ein zweiwertiges gesättigtes oder ungesättigtes aliphatisches Brückenglied mit 1–8 C-Atomen, einen Phenylen- oder Xylylenrest oder einen Rest der Formeln

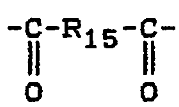 und 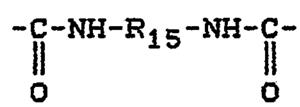

steht, wobei $R_{15}$ einen $C_1$–$C_{10}$-Alkylen-, einen Phenylen-, Toluylen- oder Naphthylenrest bezeichnet, und

A für einen Rest der Formel

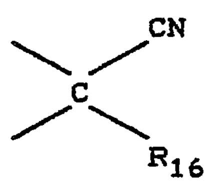

worin

$R_{16}$ Cyan, $C_1$–$C_6$-Alkoxy-$C_1$–$C_6$-alkoxycarbonyl, gegebenenfalls durch $C_1$–$C_6$-Alkyl, Benzyl, Naphthyl oder Phenyl substituiertes Carbamoyl, wobei Phenyl, Benzyl, Naphthyl, durch Chlor, Brom, $C_1$–$C_6$-Al-

kyl, $C_1$–$C_6$-Alkoxy, Nitro, Trifluormethyl, $C_1$–$C_6$-Alkylcarbonyl, Cyan, $C_1$–$C_6$-Alkylamino, Benzoylamino, Phthalimidyl, Carbamoyl, substituiert sein können, $C_1$–$C_6$-Alkylcarbonyl; Benzoyl, $C_1$–$C_6$-Alkoxycarbonyl, Benzyloxycarbonyl, Phenoxycarbonyl, wobei Benzoyl, Benzyl und Phenoxy durch Halogen, Nitro, $C_1$–$C_6$-Alkyl, $C_1$–$C_6$-Alkylcarbonylamino, Phthalimidyl, substituiert sein können oder gegebenenfalls durch Halogen, Nitro, Cyan, Trifluormethyl substituiertes Phenyl bezeichnet oder einen Rest der Formel

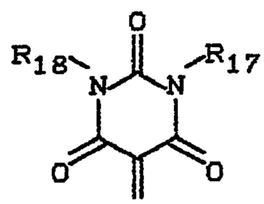

in der
$R_{17}$, $R_{18}$ Wasserstoff, $C_1$–$C_6$-Alkyl, gegebenenfalls durch Halogen, $C_1$–$C_6$-Alkyl, $C_1$–$C_6$-Alkoxy oder Nitro substituiertes Phenyl, α-Naphthyl und β-Naphthyl
bezeichnen, steht.

2. Verbindungen gemäß Anspruch 1, bei denen $R_1$, $R_2$ für

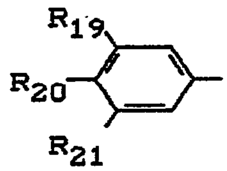

stehen, wobei
$R_{19}$, $R_{20}$, $R_{21}$ Wasserstoff, Halogen, –CN, –$R_5$, –$OR_6$, –$SR_6$–$N(R_7)(R_8)$, –$COOR_9$, –$N(R_{10})COR_9$, –$N(R_6)COOR_9$, –$N(R_6)CON(R_7)(R_8)$, –$NHSO_2R_9$, –$SO_2R_9$, –$SOR_9$, $SO_2OR_9$, –$CON(R_7)(R_8)$, –$SO_2N(R_7)(R_8)$, –N=N–$R_{11}$, –$OCOR_9$ und –$OCONHR_9$ mit den in Anspruch 1 angegebenen Bedeutungen für $R_5$, $R_6$, $R_7$, $R_8$, $R_9$, $R_{10}$ und $R_{11}$ bezeichnen.

3. Verbindungen gemäß Anspruch 1, bei denen $R_1$, $R_2$ für

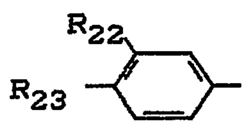

stehen, in der
$R_{22}$, $R_{23}$ Wasserstoff, Halogen, –$CF_3$, $C_1$–$C_4$-Alkyl, –CN, $C_1$–$C_4$-Alkoxy, Mono- oder Di-$C_1$–$C_4$-Alkylamino, gegebenenfalls durch Chlor substituiertes Benzoyl oder Benzoylamino, Naphthyl und Reste der Formeln

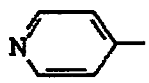 , 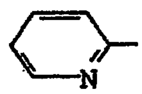 , 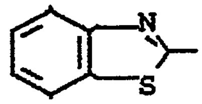 ,

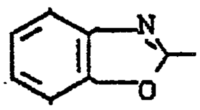 , 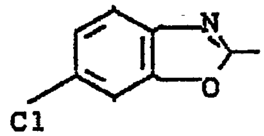 ,

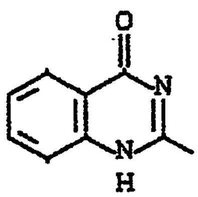 , 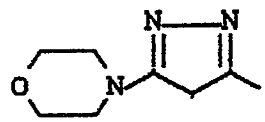

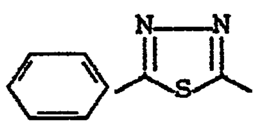 , 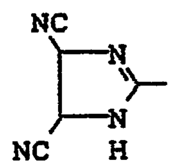 , 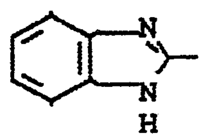 ,

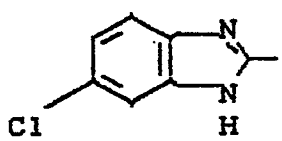

bezeichnen.

4. Verbindungen gemäß den Ansprüchen 1–3, bei denen $R_3 = R_4 = H$ ist.

5. Verbindung gemäß Anspruch 1, bei der $R_3 = R_4 = H$ und

$R_1 = R_2 =$ 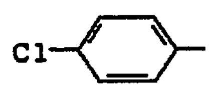

ist.

6. Verfahren zum Färben organischer makromolekularer Stoffe, dadurch gekennzeichnet, daß man heterocyclische Verbindungen gemäß den Ansprüchen 1–5 verwendet.

**Claims**

1. 2,4-Dioxopyrrolo[2,3-c]pyrroles which in one of their tautomeric forms conform to the formula

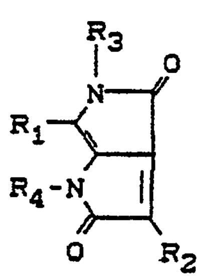 (I)

in which

$R_1$, $R_2$ signify phenyl, biphenylyl, naphthyl or a heterocyclic aromatic radical, which contains 1, 2, 3 or 4

five-, six- or seven-membered rings of which at least one contains 1, 2 or 3 hetero atoms from the series N, O, S, and wherein, the radicals mentioned for $R_1$, $R_2$ can be substituted by halogen, –CN, $-R_5$, $OR_6$, $SR_6$, $-N(R_7)(R_8)$, $-COOR_9$, $-N(R_{10})COR_9$, $-N(R_6)COOR_9$, $N(R_6)CON(R_7)(R_8)$, $-NHSO_2R_9$, $-SO_2R_9$, $-SOR_9$, $-SO_2OR_9$, $-CON(R_7)(R_8)$, $-SO_2N(R_7)(R_8)$, $-N{=}N-R_{11}$, $-OCOR_9$ and $-OCONHR_9$,
$R_3$, $R_4$ stand for hydrogen, $C_2$–$C_{18}$-alkenyl and $C_2$–$C_{18}$-alkynyl, which can each be substituted by halogen, –CN, $-OCOR_9$, $-OR_7$, $-COOR_9$, $-SR_7$, $-CON(R_7)(R_8)$ and $-OCONHR_9$, epoxyethyl, phenyl and naphthyl, which can each be substituted by F, Cl, –Br, $-NO_2$, –CN, $-CF_3$, $C_1$–$C_{18}$-alkyl, $C_1$–$C_{18}$-alkoxy, phenyl, phenoxy, $C_1$–$C_6$-alkoxycarbonyl and phenoxycarbonyl, $-R_5$, $-COR_6$, $-CON(R_7)(R_8)$,

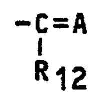

or a radical of the formula

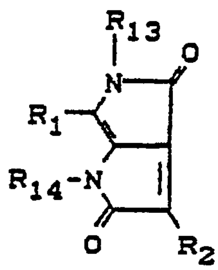

(Ia)

and
$R_5$ signifies $C_1$–$C_{18}$-alkyl or $C_3$–$C_7$-cycloalkyl which can each be substituted by halogen, –CN, $-OCOR_9$, $-OR_7$, $-COOR_9$, $-SR_7$, $-CON(R_7)(R_8)$ and $-OCONHR_9$,
$R_6$, $R_7$, $R_8$ signify hydrogen, $C_1$–$C_{18}$-alkyl, cyclopentyl and cyclohexyl which can each be substituted by Cl, Br, F, –CN, –OH, mono-$C_1$–$C_4$-alkylamino, di-$C_1$–$C_4$-alkylamino, optionally Cl-, Br-, F-, $C_1$–$C_6$-alkyl- or $C_1$–$C_6$-alkoxy-substituted phenyl or naphthyl, or by heterocyclic aromatic radicals of a 5- or 6-membered heterocyclic ring system which incorporates 1 or 2 hetero atoms from the series O, N, S and onto which a benzene ring can be fused, phenyl, naphthyl, phenyl-$C_1$–$C_4$-alkyl and naphthyl-$C_1$–$C_4$-alkyl which can each be substituted by Cl, Br, F, $C_1$–$C_{18}$-alkyl or $C_1$–$C_{18}$-alkoxy, the radical of a 5- or 6-membered heterocyclic ring which incorporates 1, 2 or 3 hetero atoms from the series O, N, S and onto which a benzene ring can be fused, or $R_7$ and $R_8$ in the group $-CON(R_7)(R_8)$ together with inclusion of the N atom form a 5- or 6-membered heterocyclic ring,
$R_9$ signifies $C_1$–$C_{18}$-alkyl, cyclopentyl, cyclohexyl, phenyl-$C_1$–$C_4$-alkyl, naphthyl-$C_1$–$C_4$-alkyl, phenyl, naphthyl, or the radical of a 5- or 6-membered heterocyclic ring which incorporates 1, 2 or 3 hetero atoms from the series O, N, S and onto which a benzene ring can be fused, it being possible for the radicals mentioned for $R_9$ to be substituted like the corresponding radicals $R_7$, $R_8$,
$R_{10}$ signifies hydrogen, $-COR_9$ or $R_7$,
$R_{11}$ signifies the radical of a coupling component from the benzene, naphthalene, acetoacetic anhydride, pyrazole or pyridone series or a phenyl radical which is optionally substituted by Cl, Br, F, $C_1$–$C_{18}$-alkyl and $C_1$–$C_{18}$-alkoxy,
$R_{12}$ signifies hydrogen, $C_1$–$C_8$-alkyl, optionally F-, Cl-, Br-, $NO_2$-, CN-, $CF_3$-, $C_1$–$C_{18}$-alkyl-, $C_1$–$C_{18}$-alkoxy-, phenyl-, phenoxy-, $C_1$–$C_6$-alkoxycarbonyl- and phenoxycarbonyl-substituted phenyl or naphthyl,
$R_{13}$ stands for hydrogen or can take on the meanings of $R_3$, $R_4$ except the radical of the formula Ia,
$R_{14}$ stands for a divalent saturated or unsaturated aliphatic bridge member having 1–8 C atoms, a phenylene or xylylene radical or a radical of the formulae

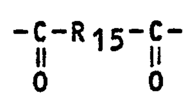 and 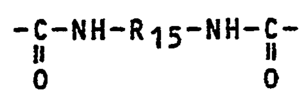

where $R_{15}$ signifies a $C_1$–$C_{10}$-alkylene, a phenylene, toluylene or naphthylene radical and
A stands for a radical of the formula

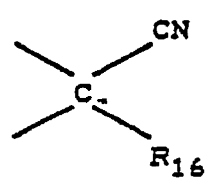

in which
$R_{16}$ denotes cyano, $C_1$–$C_6$-alkoxy-$C_1$–$C_6$-alkoxy-carbonyl, carbamoyl which is optionally substituted by $C_1$–$C_6$-alkyl, benzyl, naphthyl or phenyl, it being possible for phenyl, benzyl, naphthyl to be substituted by chlorine, bromine, $C_1$–$C_6$-alkyl, $C_1$–$C_6$-alkoxy, nitro, trifluoromethyl, $C_1$–$C_6$-alkylcarbonyl, cyano, $C_1$–$C_6$-alkylamino, benzoylamino, phthalimidyl, carbamoyl, $C_1$–$C_6$-alkylcarbonyl; benzoyl, $C_1$–$C_6$-alkoxycarbonyl, benzyloxycarbonyl, phenoxycarbonyl, it being possible for benzoyl, benzyl and phenoxy to be substituted by halogen, nitro, $C_1$–$C_6$-alkyl, $C_1$–$C_6$-alkylcarbonylamino, phthalimidyl or phenyl which is optionally substituted by halogen, nitro, cyano and trifluoromethyl or a radical of the formula

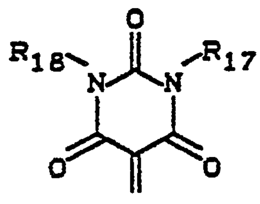

in which
$R_{17}$, $R_{18}$ signify hydrogen, $C_1$–$C_6$-alkyl, optionally halogen-, $C_1$–$C_6$-alkyl-, $C_1$–$C_6$-alkoxy- or nitro-substituted phenyl, α-naphthyl and β-napthyl.

2. Compounds according to Claim 1, in which $R_1$, $R_2$ stand for

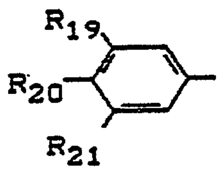

where
$R_{19}$, $R_{20}$, $R_{21}$ signify hydrogen, halogen, $-CN$, $-R_5$, $-OR_6$, $-SR_6-N(R_7)(R_8)$, $-COOR_9$, $-N(R_{10})COR_9$, $-N(R_6)COOR_9$, $-N(R_6)CON(R_7)(R_8)$, $-NHSO_2R_9$, $-SO_2R_9$, $-SOR_9$, $SO_2OR_9$, $-CON(R_7)(R_8)$, $-SO_2N(R_7)(R_8)$, $-N{=}N-R_{11}$, $-OCOR_9$ and $-OCONHR_9$ with the meanings given in Claim 1 for $R_5$, $R_6$, $R_7$, $R_8$, $R_9$, $R_{10}$ and $R_{11}$.

3. Compounds according to Claim 1, in which $R_1$, $R_2$ stand for

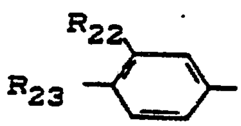

in which
$R_{22}$, $R_{23}$ signify hydrogen, halogen, $-CF_3$, $C_1$–$C_4$-alkyl, $-CN$, $C_1$–$C_4$-alkoxy, mono- or di-$C_1$–$C_4$-alkylamino, optionally chlorine-substituted benzoyl or benzoylamino, naphthyl and radicals of the formulae

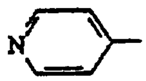 , 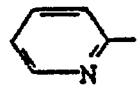 , 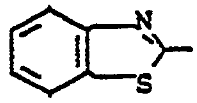 ,

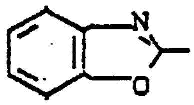 , 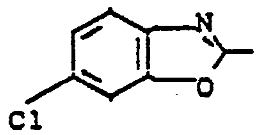 ,

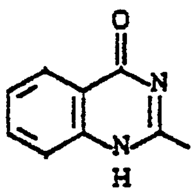 , 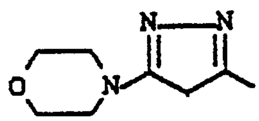

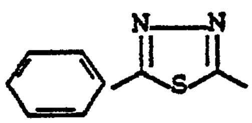 , 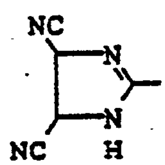 , 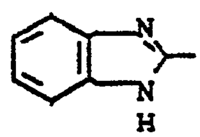 ,

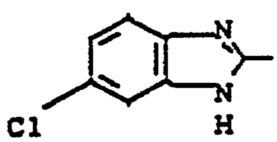

4. Compounds according to Claims 1–3 in which $R_3 = R_4 = H$.
5. Compound according to Claim 1 in which $R_3 = R_4 = H$ and

$R_1 = R_2 =$ 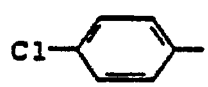

6. Process for colouring organic macromolecular materials, characterized in that heterocyclic compounds according to Claims 1–5 are used.

**Revendications**

1. 2,4-Dioxopyrrolo[2,3-c]pyrroles qui, dans l'une de leurs formes tautomères, répondent à la formule

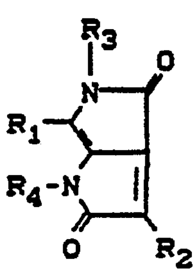

(I)

dans laquelle
$R_1$, $R_2$ représentent chacun un groupe phényle, diphénylyle, naphtyle ou un groupe aromatique-hétérocyclique qui contient 1, 2, 3 ou 4 cycles à 5, 6 ou 7 chaînons, dont l'un au moins contient 1, 2 ou 3 hétéro-atomes choisis parmi N, O, S, les substituants mentionnés pour $R_1$, $R_2$ pouvant eux-mêmes être substitués par des halogènes, des groupes $-CN$, $-R_5$, $OR_6$, $SR_6$, $-N(R_7)(R_8)$, $-COOR_9$, $-N(R_{10})COR_9$, $-N(R_6)COOR_9$, $N(R_6)CON(R_7)(R_8)$, $-NHSO_2R_9$, $-SO_2R_9$, $-SOR_9$, $-SO_2OR_9$, $-CON(R_7)(R_8)$, $-SO_2N(R_7)(R_8)$, $-N{=}N-R_{11}$, $-OCOR_9$ et $-OCONHR_9$,

$R_3$, $R_4$ représentent chacun l'hydrogène, un groupe alcényle en $C_2$–$C_{18}$ ou alcynyle en $C_2$–$C_{18}$ qui peuvent être substitués par des halogènes, des groupes $-CN$, $-OCOR_9$, $-OR_7$, $-COOR_9$, $-SR_7$, $-CON(R_7)(R_8)$, $-OCONHR_9$; un groupe époxyéthyle, phényle ou naphtyle, qui peuvent être substitués par F, Cl, Br, $-NO_2$, $-CN$, $-CF_3$, des groupes alkyle en $C_1$–$C_{18}$, alcoxy en $C_1$–$C_{18}$, phényle, phénoxy, (alcoxy en $C_1$–$C_6$)-carbonyle et phénoxycarbonyle; un groupe $-R_5$, $-COR_6$, $-CON(R_7)(R_8)$,

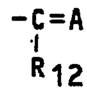

ou un groupe de formule

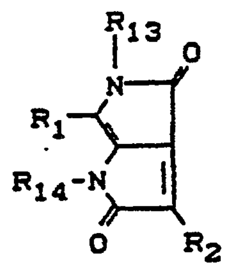

(Ia)

et

$R_5$ représente un groupe alkyle en $C_1$–$C_{18}$ ou cycloalkyle en $C_3$–$C_7$ qui peuvent être substitués par des halogènes, des groupes $-CN$, $-OCOR_9$, $-OR_7$, $-COOR_9$, $-SR_7$, $-CON(R_7)(R_8)$ et $-OCONHR_9$,

$R_6$, $R_7$, $R_8$ représentent chacun l'hydrogène, un groupe alkyle en $C_1$–$C_{18}$, cyclopentyle, cyclohexyle, qui peuvent être substitués par Cl, Br, F, des groupes $-CN$, $-OH$, mono-(alkyle en $C_1$–$C_4$)-amino, di-(alkyle en $C_1$–$C_4$)-amino, phényle ou naphtyle eux-mêmes éventuellement substitués par Cl, Br, F, des groupes alkyle en $C_1$–$C_6$ et alcoxy en $C_1$–$C_6$, ou des groupes aromatiques-hétérocycliques d'un système hétérocyclique à 5 ou 6 chaînons avec 1 ou 2 hétéroatomes choisis parmi O, N, S, sur lequel un cycle benzénique peut être condensé; un groupe phényle, naphtyle, phényl-alkyle en $C_1$–$C_4$ ou naphtyl-alkyle en $C_1$–$C_4$, qui peuvent être substitués par Cl, Br, F, des groupes alkyle en $C_1$–$C_{18}$ ou alcoxy en $C_1$–$C_{18}$, le radical d'un hétérocycle à 5 ou 6 chaînons contenant 1, 2 ou 3 hétéroatomes choisis parmi O, N, S et sur lequel un cycle benzénique peut être condensé,

ou bien $R_7$ et $R_8$ forment ensemble, dans le groupe $-CON(R_7)(R_8)$, avec l'atome d'azote, un hétérocycle à 5 ou 6 chaînons,

$R_9$ représente un groupe alkyle en $C_1$–$C_{18}$, cyclopentyle, cyclohexyle, phényl-alkyle en $C_1$–$C_4$, naphtyl-alkyle en $C_1$–$C_4$, phényle, naphtyle, ou le radical d'un hétérocycle à 5 ou 6 chaînons contenant 1, 2 ou 3 hétéroatomes choisis parmi O, N, S et sur lequel un cycle benzénique peut être condensé, les substituants mentionnés pour $R_9$ pouvant être substitués comme les groupes $R_7$, $R_8$ correspondants,

$R_{10}$ représente l'hydrogène, un groupe $-COR_9$ ou $R_7$,

$R_{11}$ représente le radical d'un copulant de la série benzénique, de la série naphtalénique, de la série de l'anhydride acétylacétique, de la série du pyrazole ou de la série de la pyridone, ou un groupe phényle éventuellement substitué par Cl, Br, F, des groupes alkyle en $C_1$–$C_{18}$ et alcoxy en $C_1$–$C_{18}$,

$R_{12}$ représente l'hydrogène, un groupe alkyle en $C_1$–$C_8$, un groupe phényle ou naphtyle éventuellement substitué par F, Cl, Br, $-NO_2$, $-CN$, $-CF_3$, des groupes alkyle en $C_1$–$C_{18}$, alcoxy en $C_1$–$C_{18}$, phényle, phénoxy, (alcoxy en $C_1$–$C_6$)-carbonyle et phénoxycarbonyle,

$R_{13}$ représente l'hydrogène, ou a les mêmes significations que $R_3$, $R_4$, à l'exception du groupe de formule Ia,

$R_{14}$ représente un pont aliphatique divalent saturé ou insaturé en $C_1$–$C_8$, un groupe phénylène ou xylylène, ou un groupe de formule

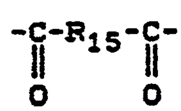 ou 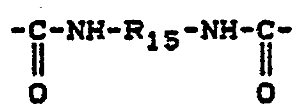

dans lequel $R_{15}$ représente un groupe alkylène en $C_1$–$C_{10}$, phénylène, toluylène ou naphtylène, et

A représente un groupe de formule

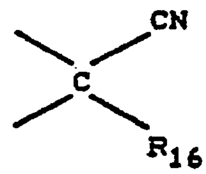

dans laquelle
$R_{16}$ représente un groupe cyano, (alcoxy en $C_1$–$C_6$)-(alcoxy en $C_1$–$C_6$)-carbonyle, carbamoyle éventuellement substitué par des groupes alkyle en $C_1$–$C_6$, benzyle, naphtyle ou phényle, ces groupes phényle, benzyle, naphtyle pouvant eux-mêmes être substitués par le chlore, le brome, des groupes alkyle en $C_1$–$C_6$, alcoxy en $C_1$–$C_6$, nitro, trifluorométhyle, (alkyle en $C_1$–$C_6$)-carbonyle, cyano, (alkyle en $C_1$–$C_6$)-amino, benzoylamino, phtalimidyle, carbamoyle, un groupe (alkyle en $C_1$–$C_6$)-carbonyle; un groupe benzoyle, (alcoxy en $C_1$–$C_6$)-carbonyle, benzyloxycarbonyle, phénoxycarbonyle, dans lesquels les groupes benzoyle, benzyle et phénoxy peuvent eux-mêmes être substitués par des halogènes, des groupes nitro, alkyle en $C_1$–$C_6$, (alkyle en $C_1$–$C_6$)-carbonylamino, phtalimidyle, ou bien un groupe phényle éventuellement substitué par des halogènes, des groupes nitro, cyano, trifluorométhyle, ou bien un groupe de formule

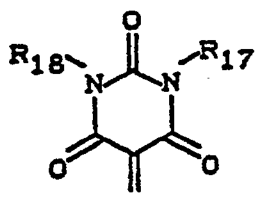

dans laquelle
$R_{17}$, $R_{18}$ représentent chacun l'hydrogène, un groupe alkyle en $C_1$–$C_6$, un groupe phényle, alpha-naphtyle ou bêta-naphtyle éventuellement substitué par des halogènes, des groupes alkyle en $C_1$–$C_6$, alcoxy en $C_1$–$C_6$, ou nitro.

2. Composés selon la revendication 1, pour lesquels $R_1$, $R_2$ représentent chacun le groupe

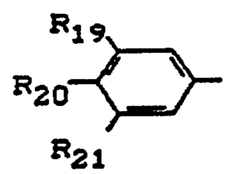

dans lequel
$R_{19}$ $R_{20}$, $R_{21}$ représentent chacun l'hydrogène, un halogène, un groupe –CN, $-R_5$, $-OR_6$, $-SR_6$ $-N(R_7)(R_8)$, $-COOR_9$, $-N(R_{10})COR_9$, $-N(R_6)COOR_9$, $-N(R_6)CON(R_7)(R_8)$, $-NHSO_2R_9$, $-SO_2R_9$, $-SOR_9$, $SO_2OR_9$, $-CON(R_7)(R_8)$, $-SO_2N-(R_7)(R_8)$, $-N{=}N-R_{11}$, $-OCOR_9$ ou $-OCONHR_9$, $R_5$, $R_6$, $R_7$, $R_8$, $R_9$, $R_{10}$ et $R_{11}$ ayant les significations indiquées dans la revendication 1.

3. Composés selon la revendication 1, dans lesquels $R_1$, $R_2$ représentent chacun le groupe

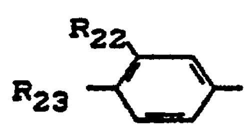

dans lequel
$R_{22}$, $R_{23}$ représentent chacun l'hydrogène, un halogène, un groupe $-CF_3$, alkyle en $C_1$–$C_4$, –CN, alcoxy en $C_1$–$C_4$, mono- ou di-(alkyle en $C_1$–$C_4$)-amino, benzoyle ou benzoylamino, naphtyle éventuellement substitués par le chlore, ou l'un des groupes de formules

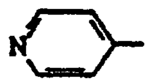 , 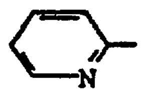 , 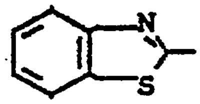 ,

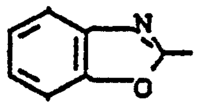 , 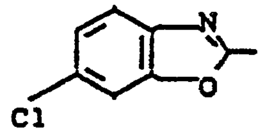 ,

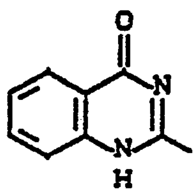 , 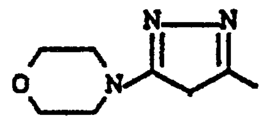

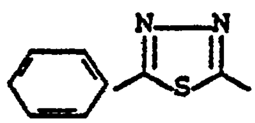 , 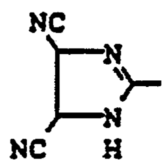 , 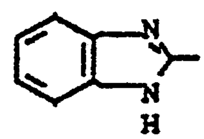 ,

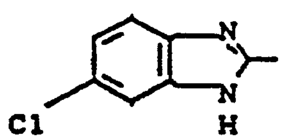

4. Composés selon les revendications 1 à 3, dans lesquels $R_3 = R_4 = H$.

5. Composé selon la revendication 1, dans lequel $R_3 = R_4 = H$, et

$R_1 = R_2 =$ 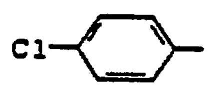 .

6. Procédé pour colorer des matières organiques macromoléculaires, caractérisé en ce que l'on utilise des composés hétérocycliques selon les revendications 1 à 5.